# FASCICULE DE BREVET EUROPEEN

(11) **EP 4 065 193 B1**
(45) Date de publication et mention de la délivrance du brevet: **08.04.2026**
(21) Numéro de dépôt: 20815883.2
(22) Date de dépôt: 26.11.2020
(51) Int. Cl.: A61M 5/142

(54) **DISPOSITIF D'INJECTION D'UN PRODUIT**
VORRICHTUNG ZUM EINSPRITZEN EINES PRODUKTES
DEVICE FOR INJECTING A PRODUCT

(30) Priorité: 26.11.2019 FR 1913273
(43) Date de publication de la demande: 05.10.2022
(73) Titulaire: NEMERA LA VERPILLIERE, 38290 La Verpillière (FR)
(72) Inventeur: PIERQUIN, Thibaut, 69300 Caluire et Cuire (FR); DIET, Sébastien, 69300 Les Houches (FR); PINTUS, Pierre, 38300 Crachier (FR); GRENOT, Gaëtan, 69004 Lyon (FR); DELVALAC, Sébastien, 69007 Lyon (FR); TODESCO, Marc, 38230 Tignieu-Jameyzieu (FR)
(74) Mandataire: LLR
(86) Numéro de dépôt international: PCT/EP2020/084528
(87) Numéro de publication internationale: WO 2021/105520

(56) Documents cités:
- WO-A1-2015/032745
- WO-A1-2016/041872
- WO-A1-2018/060027
- WO-A1-2018/219842
- WO-A1-2019/036181
- US-A1- 2019 015 582

## Description

L'invention concerne un dispositif d'injection d'un produit dans un site. L'invention concerne plus particulièrement un dispositif d'insertion d'une aiguille permettant de distribuer un produit dans le site où l'insertion de l'aiguille a été effectuée, notamment au moyen d'un cathéter (ou canule).

On connaît déjà dans l'état la technique, notamment d'après le document WO2015164645, un dispositif d'insertion d'une aiguille en vue d'une insertion de cathéter (ou canule) pour la distribution d'un médicament. Le dispositif comprend un ressort de torsion permettant un mouvement en translation verticale d'un support d'aiguille lorsque le ressort passe d'un état comprimé vers un état relâché. Le dispositif comprend en outre un système de pompe, indépendant du ressort de torsion, permettant la distribution du médicament depuis un réservoir. De plus, l'utilisation d'un ressort ou de tout autre élément d'entraînement préarmé dans ce type de dispositif, est prompt à une certaine usure des pièces plastiques avec lesquelles il est en interaction. Du fait de la robustesse nécessaire pour éviter toute activation involontaire, cette utilisation peut aussi amener des difficultés d'assemblage et/ou de fonctionnement, par exemple du point de vue de la reproductibilité des mouvements.

En effet, un élément de connexion doit tout d'abord être assemblé avec le support d'aiguille, puis l'ensemble est rapporté sur le socle tout en ayant pris soin de comprimer le ressort de torsion contre une butée prévue sur le socle et d'insérer un pion d'entraînement dans un chemin de came. Cela implique un certain nombre de contraintes d'assemblage. De plus, l'insertion de l'aiguille et la distribution du médicament sont pilotées de façon indépendante ce qui nécessite plus de composants dans le dispositif d'insertion qui présente un encombrement important.

Les documents US2019/015582 A1, WO2018/060027 A1, WO2019/036181 A1, WO2016/041872 A1, WO2015/032745 A1 et WO2018/219842 A1 décrivent d'autres dispositifs d'insertion d'une aiguille ou des dispositfs d'injection.

L'invention a notamment pour but de remédier aux inconvénients cités ci-dessus et de fournir un dispositif d'injection plus compact et plus facile d'assemblage.

A cet effet l'invention a pour objet un dispositif d'injection d'un produit dans un site comprenant :
- un corps pourvu d'une paroi d'appui sur le site,
- un support d'aiguille sur lequel est montée une aiguille, le support d'aiguille étant monté mobile par rapport à la paroi d'appui entre :
   - une position avant insertion dans laquelle l'aiguille est en retrait par rapport à la paroi d'appui,
   - au moins une position d'insertion dans laquelle l'aiguille est proéminente par rapport à la paroi d'appui, et
   - au moins une position rétractée dans laquelle l'aiguille est de nouveau en retrait par rapport à la paroi d'appui,
- un cathéter monté mobile par rapport à l'aiguille et destiné à distribuer du produit dans le site,
- des moyens d'entrainement destinés à entraîner le support d'aiguille dans un mouvement de translation entre les différentes positions du support d'aiguille,
- une pompe reliée à un réservoir de façon à transférer du produit depuis le réservoir vers le cathéter,
- un unique moteur électrique destiné à entraîner les moyens d'entrainement et à alimenter la pompe pour le transfert du produit.

Le dispositif d'injection tel que proposé par la présente invention nécessite ainsi un seul moteur qui assure l'insertion d'aiguille et l'injection de produit, ce qui permet de réduire l'encombrement du dispositif d'injection et le coût de fabrication. L'assemblage se trouve facilité grâce à un nombre de pièces réduit. En particulier, il n'est pas nécessaire de prévoir un moyen de rappel spécifique pour actionner l'insertion d'aiguille, ce qui permet de réduire le nombre de pièces promptes à l'usure. De surcroît, le pilotage du mouvement d'insertion de l'aiguille et du cathéter est plus contrôlé et aisé grâce à l'entraînement par le moteur électrique.

Suivant d'autres caractéristiques optionnelles du dispositif d'injection prises seules ou en combinaison :
- Le moteur électrique est configuré pour :
   - tourner dans un premier sens de rotation de façon à ce que les moyens d'entraînement entraînent le support d'aiguille vers l'au moins une position d'insertion :
   - tourner dans un second sens de rotation de façon à ce que la pompe soit mise en fonctionnement afin de transférer du produit depuis le réservoir vers le cathéter, et de préférence à ce que les moyens d'entraînement entraînent le support d'aiguille vers l'au moins une position rétractée, de préférence encore simultanément.

Ainsi, les différentes fonctionnalités réalisées par l'unique moteur électrique se font simplement par le changement de sens de rotation du moteur électrique, dont la commande est simple à réaliser. On comprend que le second sens de rotation correspond à un sens de rotation opposé au premier sens de rotation. Le fait que le retrait de l'aiguille et la distribution de produit se font de façon simultanée permet de rendre le dispositif d'injection plus efficace encore.
- Les moyens d'entraînement comprennent une série de dents disposées sur le pourtour d'un élément rotatif du moteur électrique et destinées à coopérer avec une crémaillère portée par au moins le support d'aiguille ou un élément d'entraînement complémentaire de préférence configuré pour être lié en translation avec le support d'aiguille.

Grâce à la coopération des dents avec la crémaillère, la rotation de l'élément rotatif est transformée facilement en translation du support d'aiguille, plus particulièrement lors de l'insertion et de la remontée de l'aiguille.

On comprend que la crémaillère peut être portée exclusivement par le support d'aiguille (par exemple l'élément d'entraînement complémentaire et le support d'aiguille sont monoblocs), ce qui permet de faire l'économie d'une pièce. L'élément d'entraînement complémentaire peut également prendre la forme d'une pièce distincte par rapport au support d'aiguille auquel cas l'utilisation d'un tel élément d'entraînement complémentaire, qui est en contact direct avec l'élément rotatif, rend possible des formes variées pour le support d'aiguille pour répondre aux éventuelles contraintes d'agencement ou d'encombrement du dispositif d'injection. Enfin, le support d'aiguille et l'élément d'entraînement complémentaire peuvent simultanément intégrer des surfaces composant la crémaillère afin de permettre un contrôle plus maîtrisé des mouvements de ces éléments dans le dispositif d'injection.
- Le dispositif d'injection comprend un support de cathéter apte à déplacer le cathéter avec l'aiguille lors du passage du support d'aiguille de sa position avant insertion à l'au moins une position d'insertion et à le désolidariser de l'aiguille de sorte qu'il reste verrouillé en mouvement lorsque le support d'aiguille passe de sa position d'insertion à l'au moins une position rétractée.
- Le dispositif d'injection comprend un moyen de verrouillage configuré pour coopérer avec des crans portés par le support de cathéter afin de verrouiller en mouvement le support de cathéter et définir au moins une première et une seconde positions d'insertion du support d'aiguille.

Le moyen de verrouillage et les crans permettent de verrouiller le support de cathéter en déplacement par rapport à la paroi d'appui à au moins deux positions différentes correspondant à au moins deux positions différentes d'insertion du support d'aiguille. Il est particulièrement intéressant de pouvoir faire varier les positions d'insertion du support d'aiguille pour faire varier la profondeur d'injection du produit, et ainsi adapter l'injection à des variables telles que la corpulence de l'utilisateur ou le type d'injection souhaitée.
- Le support de cathéter et l'élément d'entraînement complémentaire sont configurés pour être :
   - liés en translation pendant le déplacement du support d'aiguille de sa position avant insertion vers l'au moins une position d'insertion, et
   - mobiles l'un par rapport à l'autre pendant le déplacement du support d'aiguille de l'au moins une position d'insertion vers l'au moins une position rétractée.
- Le dispositif d'injection comprend un élément élastique porté par le support de cathéter ou l'élément d'entraînement complémentaire, l'élément élastique étant configuré pour maintenir l'élément d'entraînement complémentaire en contact avec les moyens d'entraînement lorsque le moteur électrique tourne dans le second sens de rotation avant insertion de l'aiguille.

L'utilisation d'un tel élément élastique rend possible une fonction d'amorçage (ou « priming » en anglais), qui permet de purger de l'air présent dans le réservoir ou en amont du cathéter, de façon à amener du produit dans le cathéter. En effet, lorsque le moteur tourne dans le second sens de rotation, en fonctionnement habituel (hors amorçage), les moyens d'entraînement entraînent le support d'aiguille vers l'au moins une position rétractée par rapport au support de cathéter.

Grâce à l'élément élastique, on peut solidariser la crémaillère avec le support de cathéter au cours de l'amorçage, avant l'insertion de l'aiguille et du cathéter dans le corps du sujet. De même, l'élément élastique amène un lien de solidarisation supplémentaire et indirect entre le support de cathéter et le support d'aiguille.

Cette configuration permet habilement de disposer d'un unique moteur pour assurer les fonctions d'insertion d'aiguille, de transfert de produit, et d'amorçage du dispositif. Avantageusement, une butée est en interaction avec l'élément élastique pour maintenir la crémaillère en interaction avec les moyens d'entraînement. L'élément élastique et la butée ne sont plus en interaction lors de la remontée de la crémaillère et du support d'aiguille après insertion. Avantageusement, l'élément élastique permet également de maintenir solidairement l'ensemble (la crémaillère, le support de cathéter et le support d'aiguille) durant des étapes de manipulation du dispositif d'injection (stockage, transport et phase d'amorçage).
- Le dispositif d'injection comprend un socle présentant un logement de guidage définissant un axe de guidage et destiné à recevoir un support d'aiguille, et dans lequel le support d'aiguille comprend des moyens de blocage configurés pour coopérer avec des moyens de blocage complémentaires portés par le logement de guidage lorsque le support d'aiguille est en position avant insertion et en l'au moins une position rétractée. Les moyens de blocage et les moyens de blocage complémentaires permettent le maintien de l'aiguille durant l'injection et de garantir la stabilité de l'aiguille lorsque le support d'aiguille n'est pas mis en mouvement, c'est-à-dire, avant l'insertion (y compris pendant l'amorçage) et après l'insertion du cathéter.
- L'élément d'entraînement complémentaire est configuré pour être lié en translation avec le support d'aiguille uniquement pendant le déplacement du support d'aiguille
   - de sa position avant insertion vers l'au moins une position d'insertion, et
   - de l'au moins une position d'insertion vers l'au moins une position rétractée,

l'élément d'entraînement complémentaire étant mobile par rapport au support d'aiguille lorsque le support d'aiguille est en position avant insertion ou en l'au moins une position rétractée.

Ceci permet la rotation des moyens d'entraînement pendant l'amorçage et l'injection, en rendant l'élément d'entrainement complémentaire non limité en mouvement par le support d'aiguille.
- L'élément d'entraînement complémentaire comprend une portion souple portant la crémaillère, de préférence la portion souple présente une forme arquée. Cette architecture et forme de l'élément d'entraînement complémentaire permet un arrangement horizontal de l'élément d'entraînement complémentaire avant et après l'insertion de l'aiguille afin de diminuer davantage la taille du dispositif d'injection.
- Les moyens d'entrainement comprennent une première série de dents disposées sur le pourtour d'un élément rotatif et destinées à coopérer avec une seconde série de dents portées par un élément rotatif complémentaire pourvu d'un chemin de came configuré pour guider le support d'aiguille en translation. Cet agencement permet de diminuer la taille du dispositif d'injection.

L'invention a également pour objet un kit d'assemblage d'un dispositif d'injection d'un produit dans un site comprenant :
- un corps pourvu d'une paroi d'appui sur le site,
- un support d'aiguille, le support d'aiguille étant monté mobile par rapport à la paroi d'appui entre :
   - une position avant insertion dans laquelle l'aiguille est en retrait par rapport à la paroi d'appui,
   - au moins une position d'insertion dans laquelle l'aiguille est proéminente par rapport à la paroi d'appui, et
   - au moins une position rétractée dans laquelle l'aiguille est de nouveau en retrait par rapport à la paroi d'appui,
- un cathéter configuré pour être mobile par rapport à l'aiguille et destiné à distribuer du produit dans le site,
- des moyens d'entrainement destinés à entraîner le support d'aiguille dans un mouvement de translation entre les différentes positions du support d'aiguille,
- une pompe reliée à un réservoir de façon à transférer du produit depuis le réservoir vers le cathéter,
- un unique moteur électrique destiné à entraîner les moyens d'entrainement et à alimenter la pompe pour le transfert du produit.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue partielle en perspective d'un dispositif d'injection selon un mode de réalisation de l'invention ;
[Fig. 2] la figure 2 est une vue éclatée d'un sous-ensemble du dispositif d'injection de la figure 1 ;
[Fig. 3] la figure 3 est un ensemble de vues en perspective (figures 3a-3e) illustrant différentes étapes de fonctionnement du sous ensemble du dispositif d'injection de la figure 2 en interaction avec des moyens d'entraînement ;
[Fig. 4] la figure 4 est un ensemble de vues en coupe et en perspective (figures 4a-4e) illustrant les étapes de fonctionnement du sous ensemble du dispositif d'injection de la figure 3.

### Description détaillée

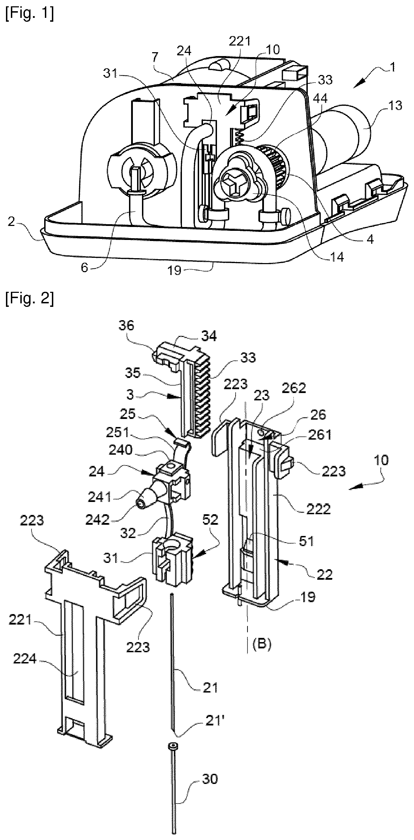

On a représenté sur les figures 1 à 4 un dispositif d'injection selon un mode de réalisation de l'invention, désigné par la référence générale 1. Tel que visible à la figure 1, le dispositif d'injection 1 comprend un corps 2 présentant une paroi d'appui 19 destinée à être positionnée en contact direct avec la peau d'un patient. Le dispositif d'injection 1 est configuré pour assurer une injection d'un produit, de préférence liquide, généralement un médicament, dans un site du patient sur une durée relativement longue, généralement de plusieurs minutes, voire de plusieurs heures.

Les produits pharmaceutiques susceptibles d'être utilisés dans le dispositif d'insertion sont par exemple les peptides, les protéines, les hormones, les principes actifs d'origine biologique, les principes actifs à base de nucléotides, les formules nutritionnelles et d'autres substances.

Ces principes actifs peuvent être, sans s'y limiter, les insulines, les analogues de l'insuline tels que l'insuline lispro ou l'insuline glargine, les dérivés de l'insuline, le C-peptide, les agonistes du récepteur GLP-1 tels que le dulaglutide ou le liraglutide, le glucagon, les analogues du glucagon, les dérivés du glucagon, les polypeptides inhibiteur gastrique (GIP), les analogues du GIP, les dérivés du GIP, les analogues de l'oxyntomoduline, les dérivés de l'oxyntomoduline, les anticorps thérapeutiques, tel que les anticorps monoclonaux et tout agent thérapeutique pouvant être délivré par le dispositif ci-dessus. Le médicament tel qu'il est utilisé dans le dispositif peut être formulé avec un ou plusieurs excipients.

Le dispositif d'injection 1 peut être à cette fin porté par l'utilisateur pendant l'injection, par exemple sur la taille.

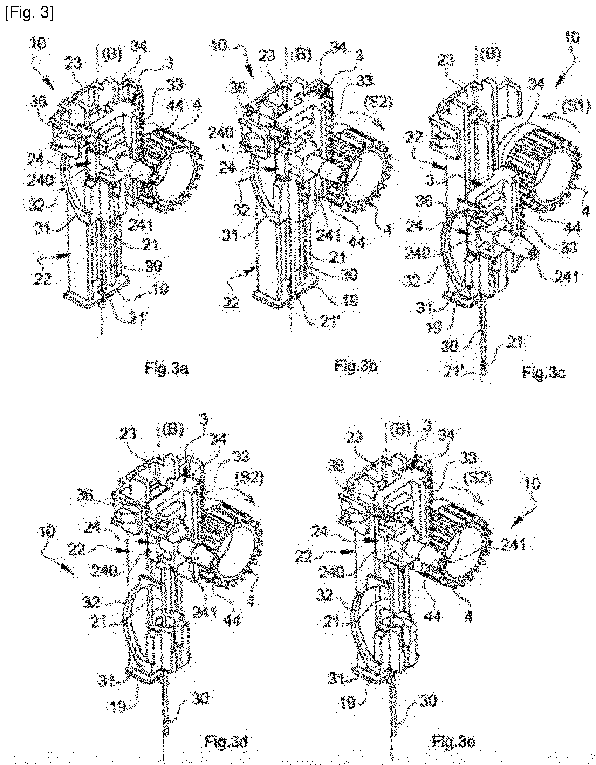

Le dispositif d'injection 1 comprend en outre, comme visible toujours à la figure 1, un moteur électrique 13 comprenant un arbre sur lequel sont montés des moyens d'entraînement 4, 44 qui consistent, dans l'exemple illustré, en un élément rotatif 4 pourvu d'une série de dents 44 réparties de façon uniforme sur le pourtour de l'élément rotatif 4. Le moteur électrique 13 est apte à tourner dans un premier sens de rotation S1 (voir par exemple figure 3c) et un second sens de rotation S2 (voir par exemple figure 3d).

Le dispositif d'injection 1 comprend encore une pompe 14, par exemple une pompe péristaltique telle qu'illustrée à la figure 1, montée coaxialement avec l'élément rotatif 4 et actionnée par le même moteur électrique 13. La pompe 14 est reliée à un réservoir 7 du produit au moyen d'une tubulure 6 flexible de façon à ce que, lorsque la pompe 14 est mise en fonctionnement, du produit soit transféré depuis le réservoir 7 vers une unité d'injection 10 qui sera décrite plus loin. La figure 1 est une vue partielle du dispositif d'injection 1 car ce dernier peut comprendre un couvercle ou boîtier permettant de former un espace fermé avec la paroi d'appui 19 afin de rendre les éléments décrits précédemment non visibles ou accessibles depuis l'extérieur du dispositif d'injection 1.

La figure 2 illustre un sous-ensemble du dispositif d'injection 1, qui est une unité d'injection 10. L'unité d'injection 10 comprend un socle 22 comprenant un premier guide vertical 221 et un second guide vertical 222 formant un logement de guidage 23 définissant un axe de guidage (B). Le premier guide vertical 221 et le second guide vertical 222 sont montés solidaires l'un par rapport à l'autre, par des moyens de clipsage 223 par exemple. L'axe de guidage (B) peut être prévu sensiblement perpendiculaire à la paroi d'appui 19 ou selon toute inclinaison adaptée. En outre, Le socle 22 comprend, sur le premier guide vertical 221, une ouverture traversante 224 ayant une forme longitudinale selon l'axe de guidage (B). Le socle 22 comprend encore un moyen de verrouillage 51 porté par le second guide vertical 222 à proximité de la paroi d'appui 19. La fonction des différents éléments portés par le socle 22 est décrite plus loin.

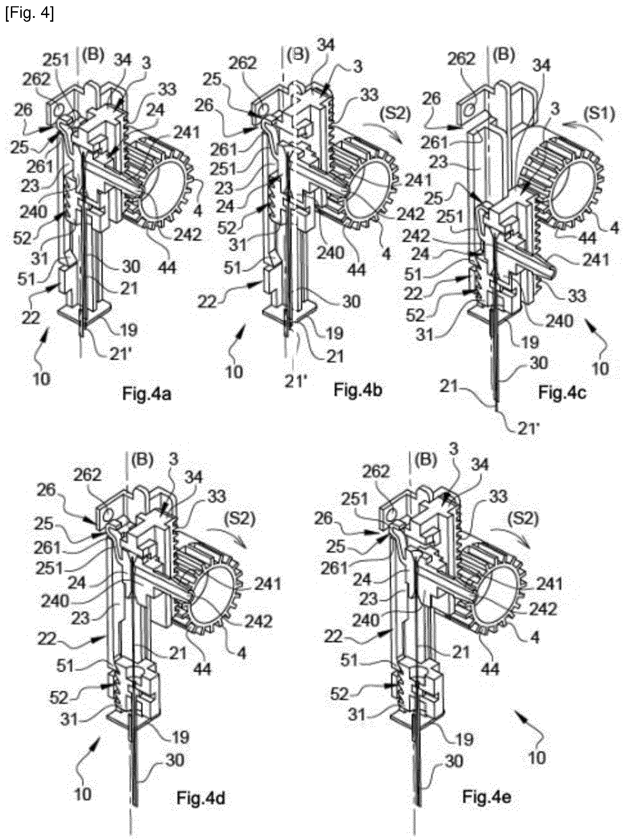

Tel que visible à la figure 2, l'unité d'injection 10 comprend un support d'aiguille 24 monté mobile dans le logement de guidage 23 entre différentes positions telles qu'illustrées aux figure 3 et 4. Le support d'aiguille 24 comprend un bloc principal 240 à section sensiblement en forme de croix selon un plan parallèle à la paroi d'appui 19. Par ailleurs, le bloc principal 240 présente une forme substantiellement complémentaire à celle du logement de guidage 23, de telle sorte que le support d'aiguille 24 puisse coulisser dans le logement de guidage 23 selon l'axe de guidage (B).

Le support d'aiguille 24 comprend en outre un moyen de blocage 25, sous forme d'un crochet élastique 251 monté sur une portion souple, le moyen de blocage 25 étant configuré pour coopérer avec des moyens de blocage complémentaires 26 comprenant une première butée verticale 261 et une seconde butée verticale 262 portées par le socle 22, la coopération entre les moyens de blocage 25 et les moyens de blocage complémentaires 26 étant mieux visible à la figure 4.

Comme montré par les figures 2 à 4, le support d'aiguille 24 comprend encore une protubérance 241 destinée à traverser l'ouverture traversante 224 longitudinale et à coulisser dans celle-ci. La protubérance 241 est destinée à être raccordée à la tubulure 6 comme illustré à la figure 1 pour être en communication fluidique avec le réservoir 7 et le produit qu'il contient. Pour ce faire, la protubérance 241 est pourvue d'un canal de distribution 242 tel qu'illustré à la figure 4 permettant de faire passer du produit dans le support d'aiguille 24. Une aiguille 21 d'insertion est montée solidaire au support d'aiguille 24 et présente un orifice latéral en communication fluidique avec le canal de distribution 242, permettant ainsi au produit à injecter d'arriver jusqu'à l'extrémité d'insertion 21' et donc au site d'injection une fois l'aiguille 21 insérée.

Selon l'invention, le support d'aiguille 24 est monté mobile par rapport à la paroi d'appui 19 entre :
- une position avant insertion dans laquelle l'aiguille 21 est en retrait par rapport à la paroi d'appui 19,
- au moins une position d'insertion dans laquelle l'aiguille 21 est proéminente par rapport à la paroi d'appui 19, et
- au moins une position rétractée dans laquelle l'aiguille 21 est de nouveau en retrait par rapport à la paroi d'appui 19.

Pour ce faire, l'unité d'injection 10 comprend un élément d'entraînement complémentaire 3 (voir par exemple figure 2) destiné à entraîner le support d'aiguille 24 dans un mouvement de translation entre les différentes positions du support d'aiguille 24. Tel que visible aux figures 2 à 4, l'élément d'entraînement complémentaire 3 présente une forme en L et comprend une première portion 34 qui est horizontale, c'est-à-dire qui s'étend selon un axe perpendiculaire à l'axe de guidage (B), et une seconde portion 35 verticale, c'est-à-dire qui s'étend selon un axe parallèle à l'axe de guidage (B). La première portion 34 présente une surface plane telle que visible à la figure 4 destinée à entrer en contact avec le support d'aiguille 24 et à entraîner le support d'aiguille 24 en mouvement. La première portion 34 comprend en outre un pion de verrouillage 36 (voir par exemple figure 3a) agencé à une extrémité de la première portion 34 opposée à la seconde portion 35. La seconde portion 35 est pourvue d'une crémaillère 33 (voir par exemple figure 2) destinée à coopérer avec la série de dents 44 sur l'élément rotatif 4 afin que l'élément d'entraînement complémentaire 3 puisse être entraîné en mouvement de translation selon l'axe de guidage (B) dans les deux directions opposées l'une à l'autre et selon les premier et second sens de rotation S1, S2 du moteur électrique 13.

L'unité d'injection 10 comprend encore un cathéter 30 porté par un support de cathéter 31, tel que visible aux figures 2 à 4. Dans l'exemple illustré, notamment aux figures 2 et 3, le support de cathéter 31 comprend un élément élastique 32 ayant une forme d'arc, dont une extrémité est solidaire du support de cathéter 31 et l'autre extrémité forme une butée destinée à coopérer avec le pion de verrouillage 36 pour maintenir la crémaillère 33 en interaction avec la série de dents 44. Avantageusement, l'élément élastique 32 et le pion de verrouillage 36 ne sont plus en interaction lors de la remontée de la crémaillère 33 et du support d'aiguille 24 après insertion de l'aiguille 21 et rotation du moteur électrique 13 selon le second sens de rotation S2 (voir figures 3d et 3e).

Le support de cathéter 31 est lié en translation au support d'aiguille 24 pendant l'insertion de l'aiguille 21. Le cathéter 30 est monté mobile par rapport à l'aiguille 21. De même, l'aiguille 21 étant logée à l'intérieur du cathéter 30, l'insertion de l'aiguille 21 dans le site permet donc l'insertion du cathéter 30 de façon simultanée.

Les figures 3 et 4 illustrent les différentes étapes de fonctionnement du dispositif d'injection 1 qui seront décrites comme suit : lorsque le dispositif d'injection 1 est prêt à être utilisé par un patient ou par une personne du corps médical, le support d'aiguille 24 est dans une position avant insertion dans laquelle l'aiguille 21 est en retrait par rapport à la paroi d'appui 19. Le support d'aiguille 21 est maintenu à cette position avant insertion par l'interaction entre le moyen de blocage 25 ici sous la forme d'un crochet élastique 251 et les moyens de blocage complémentaires 26 (figures 3a et 4a). Le support de cathéter 31 et l'élément d'entrainement complémentaire 3 sont également maintenus à cette position avant insertion car ils sont respectivement en butée contre le support d'aiguille 21 d'un côté et bloqués en translation par l'interaction entre l'élément élastique 32 et le pion de verrouillage 36 porté par l'élément d'entraînement complémentaire 3 de l'autre.

Lorsque l'utilisateur positionne le dispositif d'injection 1 sur un site à injecter, la paroi d'appui 19 est par exemple en contact direct avec le site. L'utilisateur active ensuite le moteur électrique 13, au moyen d'un bouton de commande par exemple, pour mettre en rotation le moteur électrique 13 ainsi que l'élément rotatif 4 dans le second sens de rotation S2 comme visible aux figures 3b et 4b. Le dispositif d'injection 1 est alors en phase d'amorçage, au cours de laquelle la pompe 14 commence à transférer du produit depuis le réservoir vers le cathéter 30, successivement via le tube 6, la pompe 13 et l'aiguille 21. Cette phase d'amorçage est programmée à durer pendant un lapse de temps court, mais suffisant pour purger de l'air présent en amont du cathéter 30.

Pendant la phase d'amorçage, la série de dents 44 portées par l'élément rotatif 4 étant en contact avec la crémaillère 33 portée par l'élément d'entraînement complémentaire 3 entraîne ce dernier dans une direction d'ascension par rapport à l'axe de guidage (B), c'est-à-dire dans une direction opposée à la paroi d'appui 19. Simultanément, l'élément élastique 32 applique une force sur le pion 36 vers le bas, c'est-à-dire dans une direction descendante par rapport à l'axe de guidage (B) et donc vers la paroi d'appui 19.
Ainsi, l'élément d'entraînement complémentaire 3 exerce un mouvement très faible de va-et-vient ce qui permet de maintenir en interaction l'élément d'entraînement complémentaire 3 avec la série de dents 44 de l'élément rotatif 4. L'élément d'entraînement complémentaire 3 est ainsi ramené à l'entraînement exercé via la rotation de l'élément rotatif 4 dans le second sens de rotation S2 et ainsi continuer la phase d'amorçage du dispositif d'injection 1.

A la fin de la phase d'amorçage du dispositif d'injection 1, le moteur électrique 13 tourne dans un premier sens de rotation S1, qui est opposé au second sens de rotation S2, pour entraîner le support d'aiguille 24 vers une position d'insertion prédéterminée. Plus précisément, la série de dents 44 s'engrènent avec la crémaillère 33 et transforme la rotation de l'élément rotatif 4 en translation de l'élément d'entraînement complémentaire 3 vers la paroi d'appui 19. L'élément d'entraînement complémentaire 3 entraîne lui-même le support d'aiguille 24 ainsi que le support cathéter 31 dans la même direction vers la paroi d'appui 19 comme visible aux figures 3c et 4c. Lorsque le support d'aiguille 24 sort de sa position avant insertion et se déplace vers la paroi d'appui 19, les moyens de blocage 25 ne sont alors plus en interaction avec les moyens de blocage complémentaires 26, du fait que les moyens de blocage 25 comprennent un crochet élastique 251 qui est contraint complètement dans le logement de guidage 23 tel qu'illustré par la figure 4c.

Lorsque le support d'aiguille 24 arrive à proximité de la paroi d'appui 19, les crans 52 portés par le support de cathéter 31 commencent à entrer en contact avec les moyens de verrouillage 51 l'un après l'autre. Si le support d'aiguille 24 atteint la position d'insertion prédéterminée, le cran correspondant qui est déjà en contact avec le moyen de verrouillage 51, continue à coopérer avec le moyen de verrouillage 51 pour maintenir le support de cathéter 31 en position. Si le support d'aiguille 24 n'a pas atteint la position d'insertion prédéterminée, la crémaillère 33 continue à se déplacer jusqu'à ce que le support d'aiguille 24 arrive à la position d'insertion prédéterminée. Les figures 4c à 4e illustrent une position d'insertion à laquelle le dernier cran qui entre en contact avec le moyen de verrouillage 51 coopère avec ces derniers pour maintenir le support de cathéter 31 à une position qui correspond à une position d'insertion « peu profonde » de l'aiguille 21, équivalent par exemple à la couche sous-cutanée de la peau du patient. Selon l'exemple illustré, le support d'aiguille 24 peut avoir jusqu'à 5 positions d'insertion différentes.

Une fois que le cathéter 30 est inséré dans le site à l'aide de l'insertion de l'aiguille 21, le support d'aiguille 24 se désolidarise avec le support de cathéter 31 et passe de sa position d'insertion à sa position rétractée. Pour ce faire, le moteur électrique 13 tourne à nouveau dans le second sens de rotation S2 pour que les moyens d'entraînement 4, 44 entraînent l'élément d'entraînement complémentaire 3 et le support d'aiguille 24 dans une direction d'ascension par rapport à l'axe de guidage (B), vers la position rétractée du support d'aiguille 24. La force d'entraînement du moteur électrique 13 étant plus importante que l'encliquetage entre les crans 52 et les moyens de verrouillage 51, la coopération entre l'élément élastique 32 et le pion de verrouillage 36 est rompue et l'élément d'entraînement complémentaire 3 se désolidarise du support de cathéter 31. Le support d'aiguille 24 est lié en translation avec l'élément d'entraînement complémentaire 3 grâce au crochetage formé par les moyens de blocage 25 via le crochet élastique 251 sur la première portion 34 horizontale de l'élément d'entraînement complémentaire 3.

Pendant le retrait de l'aiguille 21, le moteur électrique 13 tourne dans le second sens de rotation S2 et met donc en fonctionnement la pompe 14 permettant de transférer une quantité prédéterminée du produit depuis le réservoir 7 vers le cathéter 30.

Lorsque le support d'aiguille 24 arrive en position rétractée, le crochet élastique 251 et les moyens de blocage complémentaires 26 coopèrent à nouveau pour verrouiller le support d'aiguille 24 à cette position rétractée comme visible aux figure 3d et 4d. Le crochet élastique 251 n'étant plus en contact avec la première portion 34 horizontale de l'élément d'entraînement complémentaire 3, ce dernier devient mobile par rapport au support d'aiguille 24. Le support d'aiguille 24 est alors stable en position rétractée où il est maintenu. Ainsi, le moteur électrique 13 est capable de continuer à tourner dans le second sens de rotation S2 afin de finaliser le transfert de la quantité prédéterminée du produit.

L'assemblage du dispositif d'injection 1 est par exemple réalisé au moyen d'un kit d'assemblage comprenant :
- un corps 2 pourvu d'une paroi d'appui 19 sur le site,
- un support d'aiguille 24, le support d'aiguille 24 étant monté mobile par rapport à la paroi d'appui 19 entre :
   - une position avant insertion dans laquelle l'aiguille 21 est en retrait par rapport à la paroi d'appui 19,
   - au moins une position d'insertion dans laquelle l'aiguille 21 est proéminente par rapport à la paroi d'appui 19, et
   - au moins une position rétractée dans laquelle l'aiguille 21 est de nouveau en retrait par rapport à la paroi d'appui 19,
- un cathéter 30 configuré pour être mobile par rapport à l'aiguille 21 et destiné à distribuer du produit dans le site,
- des moyens d'entrainement 4, 44 destinés à entraîner le support d'aiguille 24 dans un mouvement de translation entre les différentes positions du support d'aiguille 24,
- une pompe 14 reliée à un réservoir de façon à transférer du produit depuis le réservoir vers le cathéter 30,
- un unique moteur électrique 13 destiné à entraîner les moyens d'entrainement 4, 44 et à alimenter la pompe 14 pour le transfert du produit.

L'invention n'est pas limitée aux modes de réalisation présentés et d'autres modes de réalisation apparaîtront clairement à l'homme du métier.

Il est notamment possible que l'élément d'entraînement complémentaire 3 comprenne une portion souple portant la crémaillère 33 souple, la portion souple présentant de préférence une forme arquée.

Il est également envisageable que les moyens d'entraînement comprennent une première série de dents disposées sur le pourtour d'un élément rotatif et destinées à coopérer avec seconde série de dents portées par un élément rotatif complémentaire pourvu d'un chemin de came configuré pour guider le support d'aiguille 24 en translation.

## Revendications

1. Dispositif d'injection (1) d'un produit dans un site comprenant :
- un corps (2) pourvu d'une paroi d'appui (19) sur le site,
- un support d'aiguille (24) sur lequel est montée une aiguille (21), le support d'aiguille (24) étant monté mobile par rapport à la paroi d'appui (19) entre :
• une position avant insertion dans laquelle l'aiguille (21) est en retrait par rapport à la paroi d'appui (19),
• au moins une position d'insertion dans laquelle l'aiguille (21) est proéminente par rapport à la paroi d'appui (19), et
• au moins une position rétractée dans laquelle l'aiguille (21) est de nouveau en retrait par rapport à la paroi d'appui (19),
- un cathéter (30) monté mobile par rapport à l'aiguille (21) et destiné à distribuer du produit dans le site,
- des moyens d'entrainement (4, 44) destinés à entraîner le support d'aiguille (24) dans un mouvement de translation entre les différentes positions du support d'aiguille (24),
- une pompe (14) reliée à un réservoir de façon à transférer du produit depuis le réservoir vers le cathéter (30), **caractérisé en ce que** le dispositif d'injection (1) comprend :
- un unique moteur électrique (13) destiné à entraîner les moyens d'entrainement (4, 44) et à alimenter la pompe (14) pour le transfert du produit.

2. Dispositif d'injection (1) selon la revendication précédente dans lequel le moteur électrique (13) est configuré pour :
- tourner dans un premier sens de rotation (S1) de façon à ce que les moyens d'entraînement (4, 44) entraînent le support d'aiguille (24) vers l'au moins une position d'insertion ;
- tourner dans un second sens de rotation (S2) de façon à ce que la pompe (14) soit mise en fonctionnement afin de transférer du produit depuis le réservoir vers le cathéter (30), et de préférence à ce que les moyens d'entraînement (4,44) entraînent le support d'aiguille (24) vers l'au moins une position rétractée, de préférence encore simultanément.

3. Dispositif d'injection (1) selon l'une quelconque des revendications précédentes dans lequel les moyens d'entraînement (4, 44) comprennent une série de dents (44) disposées sur le pourtour d'un élément rotatif (4) du moteur électrique (13) et destinées à coopérer avec une crémaillère (33) portée par au moins le support d'aiguille (24) ou un élément d'entraînement complémentaire (3) de préférence configuré pour être lié en translation avec le support d'aiguille (24).

4. Dispositif d'injection (1) selon l'une quelconque des revendications précédentes comprenant un support de cathéter (31) apte à déplacer le cathéter (30) avec l'aiguille (21) lors du passage du support d'aiguille (24) de sa position avant insertion à l'au moins une position d'insertion et à le désolidariser de l'aiguille (21) de sorte qu'il reste verrouillé en mouvement lorsque le support d'aiguille (24) passe de l'au moins une position d'insertion à l'au moins une position rétractée.

5. Dispositif d'injection (1) selon la revendication précédente comprenant un moyen de verrouillage (51) configuré pour coopérer avec des crans (52) portés par le support de cathéter (31) afin de verrouiller en mouvement le support de cathéter (31) et définir au moins une première et une seconde positions d'insertion du support d'aiguille (24).

6. Dispositif d'injection (1) selon les revendications 3 et 4, dans lequel le support de cathéter (31) et l'élément d'entraînement complémentaire (3) sont configurés pour être :
- liés en translation pendant le déplacement du support d'aiguille (24) de sa position avant insertion vers l'au moins une position d'insertion, et
- mobiles l'un par rapport à l'autre pendant le déplacement du support d'aiguille (24) de l'au moins une position d'insertion vers l'au moins une position rétractée.

7. Dispositif d'injection (1) selon l'une quelconque des revendications 4 ou 5 comprenant un élément élastique (32) porté par le support de cathéter (31) ou l'élément d'entraînement complémentaire (3), l'élément élastique (32) étant configuré pour maintenir l'élément d'entraînement complémentaire (3) en contact avec les moyens d'entraînement (4, 44) lorsque le moteur électrique (13) tourne dans le second sens de rotation (S2) avant insertion de l'aiguille (21).

8. Dispositif d'injection (1) selon l'une quelconque des revendications précédentes, comprenant un socle (22) présentant un logement de guidage (23) définissant un axe de guidage (B) et destiné à recevoir un support d'aiguille (24), et dans lequel le support d'aiguille (24) comprend des moyens de blocage (25) configurés pour coopérer avec des moyens de blocage complémentaires (26) portés par le logement de guidage (23) lorsque le support d'aiguille (24) est en position avant insertion et en l'au moins une position rétractée.

9. Dispositif d'injection (1) selon l'une quelconque des revendications précédentes combinée avec la revendication 3, dans lequel l'élément d'entraînement complémentaire (3) est configuré pour être lié en translation avec le support d'aiguille (24) uniquement pendant le déplacement du support d'aiguille (24)
- de sa position avant insertion vers l'au moins une position d'insertion, et
- de l'au moins une position d'insertion vers l'au moins une position rétractée,
l'élément d'entraînement complémentaire (3) étant mobile par rapport au support d'aiguille (24) lorsque le support d'aiguille (24) est en position avant insertion ou en l'au moins une position rétractée.

10. Kit d'assemblage d'un dispositif d'injection (1) d'un produit dans un site comprenant :
- un corps (2) pourvu d'une paroi d'appui (19) sur le site,
- un support d'aiguille (24), le support d'aiguille (24) étant monté mobile par rapport à la paroi d'appui (19) entre :
• une position avant insertion dans laquelle l'aiguille (21) est en retrait par rapport à la paroi d'appui (19),
• au moins une position d'insertion dans laquelle l'aiguille (21) est proéminente par rapport à la paroi d'appui (19), et
• au moins une position rétractée dans laquelle l'aiguille (21) est de nouveau en retrait par rapport à la paroi d'appui (19),
- un cathéter (30) configuré pour être mobile par rapport à l'aiguille (21) et destiné à distribuer du produit dans le site,
- des moyens d'entrainement (4, 44) destinés à entraîner le support d'aiguille (24) dans un mouvement de translation entre les différentes positions du support d'aiguille (24),
- une pompe (14) reliée à un réservoir de façon à transférer du produit depuis le réservoir vers le cathéter (30),
- un unique moteur électrique (13) destiné à entraîner les moyens d'entrainement (4, 44) et à alimenter la pompe (14) pour le transfert du produit.

## Patentansprüche

1. Vorrichtung (1) zum Injizieren eines Produkts an einer Stelle, aufweisend:
- einen Körper (2), der mit einer Stützwand (19) an der Stelle versehen ist,
- einen Nadelhalter (24), auf dem eine Nadel (21) angebracht ist, wobei der Nadelhalter (24) relativ zur Stützwand (19) beweglich zwischen folgenden Positionen angebracht ist:
• einer Position vor dem Einführen, in der die Nadel (21) gegenüber der Stützwand (19) zurückgesetzt ist,
• mindestens einer Position zum Einführen, in der die Nadel (21) gegenüber der Stützwand (19) hervorsteht, und
• mindestens einer zurückgezogenen Position, in der die Nadel (21) wieder gegenüber der Stützwand (19) zurückgesetzt ist,
- einen Katheter (30), der relativ zur Nadel (21) beweglich angebracht ist und dazu bestimmt ist, das Produkt an der Stelle abzugeben,
- Antriebsmittel (4, 44), die dazu bestimmt sind, den Nadelhalter (24) in einer Translationsbewegung zwischen den verschiedenen Positionen des Nadelhalters (24) anzutreiben,
- eine Pumpe (14), die mit einem Behälter verbunden ist, um das Produkt aus dem Behälter zum Katheter (30) zu befördern, **dadurch gekennzeichnet, dass** die Injektionsvorrichtung (1) aufweist:
- einen einzigen Elektromotor (13) zum Antreiben der Antriebsmittel (4, 44) und zum Antreiben der Pumpe (14) für die Beförderung des Produkts.

2. Injektionsvorrichtung (1) nach dem vorhergehenden Anspruch, wobei der Elektromotor (13) konfiguriert ist, um:
- sich in einer ersten Drehrichtung (S1) zu drehen, so dass die Antriebsmittel (4, 44) den Nadelhalter (24) in mindestens eine Einführposition treiben;
- sich in einer zweiten Drehrichtung (S2) zu drehen, so dass die Pumpe (14) in Betrieb gesetzt wird, um das Produkt aus dem Behälter zum Katheter (30) zu befördern, und vorzugsweise, dass die Antriebsmittel (4, 44) den Nadelhalter (24) in die mindestens eine zurückgezogene Position antreiben, vorzugsweise gleichzeitig.

3. Injektionsvorrichtung (1) nach einem der vorhergehenden Ansprüche, wobei die Antriebsmittel (4, 44) eine Reihe von Zähnen (44) aufweisen, die am Umfang eines rotierenden Elements (4) des Elektromotors (13) angeordnet sind und dazu bestimmt sind, mit einer Zahnstange (33) zusammenzuwirken, die von mindestens dem Nadelhalter (24) oder einem zusätzlichen Antriebselement (3) getragen wird, das vorzugsweise konfiguriert ist, um in Translationsbewegung mit dem Nadelhalter (24) verbunden zu sein.

4. Injektionsvorrichtung (1) nach einem der vorhergehenden Ansprüche, aufweisend einen Katheterhalter (31), der in der Lage ist, den Katheter (30) mit der Nadel (21) beim Durchlaufen des Nadelhalters (24) aus seiner Position vor dem Einführen in die mindestens eine Einführposition zu bewegen und ihn von der Nadel (21) zu trennen, so dass er in seiner Bewegung verriegelt bleibt, wenn der Nadelhalter (24) aus der mindestens einen Einführposition in die mindestens eine zurückgezogene Position bewegt wird.

5. Injektionsvorrichtung (1) nach dem vorhergehenden Anspruch, aufweisend ein Verriegelungsmittel (51), das konfiguriert ist, mit Rastnasen (52) am Katheterhalter (31) zusammenzuwirken, um den Katheterhalter (31) bewegungsmäßig zu verriegeln und mindestens eine erste und eine zweite Einführposition des Nadelhalters (24) zu definieren.

6. Injektionsvorrichtung (1) nach den Ansprüchen 3 und 4, wobei der Katheterhalter (31) und das zusätzliche Antriebselement (3) so konfiguriert sind, dass sie:
- während der Bewegung des Nadelhalters (24) von seiner Position vor dem Einführen in die mindestens eine Einführposition translatorisch verbunden sind und
- während der Bewegung des Nadelhalters (24) von der mindestens einen Einführposition in die mindestens eine zurückgezogene Position relativ zueinander beweglich sind.

7. Injektionsvorrichtung (1) nach einem der Ansprüche 4 oder 5, aufweisend ein elastisches Element (32), das von dem Katheterhalter (31) oder dem zusätzlichen Antriebselement (3) getragen wird, wobei das elastische Element (32) so konfiguriert ist, dass es das zusätzliche Antriebselement (3) in Kontakt mit den Antriebsmitteln (4, 44) hält, wenn der Elektromotor (13) vor dem Einführen der Nadel (21) in der zweiten Drehrichtung (S2) dreht.

8. Injektionsvorrichtung (1) nach einem der vorhergehenden Ansprüche, mit einem Sockel (22), der eine Führungsaufnahme (23) aufweist, die eine Führungsachse (B) definiert und zur Aufnahme eines Nadelhalters (24) bestimmt ist, wobei der Nadelhalter (24) Verriegelungsmittel (25) aufweist, die konfiguriert sind, mit zusätzlichen Verriegelungsmitteln (26) zusammenzuwirken, die von der Führungsaufnahme (23) getragen werden, wenn sich der Nadelhalter (24) in der Position vor dem Einführen und in mindestens einer zurückgezogenen Position befindet.

9. Injektionsvorrichtung (1) nach einem der vorhergehenden Ansprüche in Kombination mit Anspruch 3, wobei das zusätzliche Antriebselement (3) so konfiguriert ist, dass es nur während der Bewegung des Nadelhalters (24)
- von seiner Position vor dem Einführen in die mindestens eine Einführposition, und
- von der mindestens einen Einführposition in die mindestens eine zurückgezogene Position
translatorisch mit diesem verbunden ist,
wobei das zusätzliche Antriebselement (3) relativ zum Nadelhalter (24) beweglich ist, wenn sich der Nadelhalter (24) in der Position vor dem Einführen oder in der mindestens einen zurückgezogenen Position befindet.

10. Bausatz zum Zusammenbau einer Vorrichtung (1) zum Injizieren eines Produkts an einer Stelle, aufweisend:
- einen Körper (2) mit einer Stützwand (19) an der Stelle versehen ist,
- einen Nadelhalter (24), wobei der Nadelhalter (24) relativ zur Stützwand (19) beweglich zwischen folgenden Positionen angebracht ist:
• einer Position vor dem Einführen, in der die Nadel (21) gegenüber der Stützwand (19) zurückgesetzt ist,
• mindestens einer Position zum Einführen, in der die Nadel (21) gegenüber der Stützwand (19) hervorsteht, und
• mindestens einer zurückgezogenen Position, in der die Nadel (21) wieder gegenüber der Stützwand (19) zurückgesetzt ist,
- einen Katheter (30), der konfiguriert ist, relativ zur Nadel (21) beweglich zu sein und dazu bestimmt ist, das Produkt an der Stelle abzugeben,
- Antriebsmittel (4, 44), die dazu bestimmt sind, den Nadelhalter (24) in einer Translationsbewegung zwischen den verschiedenen Positionen des Nadelhalters (24) anzutreiben,
- eine Pumpe (14), die mit einem Behälter verbunden ist, um das Produkt aus dem Reservoir zum Katheter (30) zu befördern,
- einen einzigen Elektromotor (13) zum Antreiben der Antriebsmittel (4, 44) und zum Antreiben der Pumpe (14) für die Beförderung des Produkts.

## Claims

1. Injection device (1) for injecting a product into a site, comprising:
- a body (2) provided with a bearing wall (19) for bearing on the site,
- a needle support (24) on which a needle (21) is mounted, the needle support (24) being mounted so as to be able to move relative to the bearing wall (19) between:
• a pre-insertion position in which the needle (21) is retracted with respect to the bearing wall (19),
• at least one insertion position in which the needle (21) protrudes with respect to the bearing wall (19), and
• at least one retracted position in which the needle (21) is once again retracted with respect to the bearing wall (19),
- a catheter (30) mounted so as to be movable with respect to the needle (21) and intended to dispense product into the site,
- drive means (4, 44) intended to drive the needle support (24) in translation between the various positions of the needle support (24),
- a pump (14) connected to a reservoir so as to transfer product from the reservoir to the catheter (30),
**characterised in that** the injection device (1) comprises:
- a single electric motor (13) intended to drive the drive means (4, 44) and to power the pump (14) to transfer the product.

2. Injection device (1) according to the preceding claim, wherein the electric motor (13) is configured to:
- rotate in a first direction of rotation (S1) so that the drive means (4, 44) drive the needle support (24) towards the at least one insertion position;
- rotate in a second direction of rotation (S2) so that the pump (14) operates in order to transfer product from the reservoir to the catheter (30), and preferably so that the drive means (4, 44) drive the needle support (24) towards the at least one retracted position, preferably simultaneously.

3. Injection device (1) according to any one of the preceding claims, wherein the drive means (4, 44) comprise a series of teeth (44) arranged on the circumference of a rotating element (4) of the electric motor (13) and intended to cooperate with a rack (33) carried by at least the needle support (24) or a complementary drive element (3) preferably configured to be connected in translation with the needle support (24).

4. Injection device (1) according to any one of the preceding claims, comprising a catheter support (31) adapted to move the catheter (30) with the needle (21) when the needle support (24) changes from its pre-insertion position to the at least one insertion position and to separate it from the needle (21) so that its movement remains locked when the needle support (24) changes from the at least one insertion position to the at least one retracted position.

5. Injection device (1) according to the preceding claim, comprising a locking means (51) configured to cooperate with notches (52) carried by the catheter support (31) in order to lock the movement of the catheter support (31) and define at least first and second insertion positions of the needle support (24).

6. Injection device (1) according to claims 3 and 4, wherein the catheter support (31) and the complementary drive element (3) are configured to be:
- connected in translation when moving the needle support (24) from its pre-insertion position to the at least one insertion position, and
- movable with respect to each other when moving the needle support (24) from the at least one insertion position to the at least one retracted position.

7. Injection device (1) according to claim 4 or 5, comprising an elastic element (32) carried by the catheter support (31) or the complementary drive element (3), the elastic element (32) being configured to keep the complementary drive element (3) in contact with the drive means (4, 44) when the electric motor (13) rotates in the second direction of rotation (S2) before inserting the needle (21).

8. Injection device (1) according to any one of the preceding claims, comprising a base (22) having a guide housing (23) defining a guide axis (B) and intended to receive a needle support (24), and in which the needle support (24) comprises blocking means (25) configured to cooperate with complementary blocking means (26) carried by the guide housing (23) when the needle support (24) is in the pre-insertion position and in the at least one retracted position.

9. Injection device (1) according to any one of the preceding claims combined with claim 3, wherein the complementary drive element (3) is configured to be connected in translation with the needle support (24) only when moving the needle support (24)
- from its pre-insertion position to the at least one insertion position, and
- from the at least one insertion position to the at least one retracted position, the complementary drive element (3) being movable with respect to the needle support (24) when the needle support (24) is in the pre-insertion position or in the at least one retracted position.

10. Kit for assembling an injection device (1) for injecting a product into a site comprising:
- a body (2) provided with a bearing wall (19) for bearing on the site,
- a needle support (24), the needle support (24) being mounted so as to be movable with respect to the bearing wall (19) between:
• a pre-insertion position in which the needle (21) is retracted with respect to the bearing wall (19),
• at least one insertion position in which the needle (21) protrudes with respect to the bearing wall (19), and
• at least one retracted position in which the needle (21) is once again retracted with respect to the bearing wall (19),
- a catheter (30) configured so as to be movable with respect to the needle (21) and intended to dispense product into the site,
- drive means (4, 44) intended to drive the needle support (24) in translation between the various positions of the needle support (24),
- a pump (14) connected to a reservoir so as to transfer product from the reservoir to the catheter (30),
- a single electric motor (13) intended to drive the drive means (4, 44) and to power the pump (14) to transfer the product.
